(19) Europäisches Patentamt

European Patent Office

Office européen des brevets

(11) EP 0 451 811 B1

(12) EUROPEAN PATENT SPECIFICATION

(45) Date of publication and mention
of the grant of the patent:
**24.07.1996 Bulletin 1996/30**

(51) Int Cl.$^6$: **C12Q 1/00**, C12M 1/40,
G01N 33/66

(21) Application number: **91105679.4**

(22) Date of filing: **10.04.1991**

(54) **Method and apparatus for analyzing starch and other carbohydrates**

Verfahren und Gerät zur Analyse von Stärke und sonstigen Kohlenhydraten

Méthode et appareil pour analyser amidon et autres hydrates de carbons

(84) Designated Contracting States:
**DE GB**

(30) Priority: **10.04.1990 JP 95953/90**

(43) Date of publication of application:
**16.10.1991 Bulletin 1991/42**

(73) Proprietor: **NEW OJI PAPER CO., LTD.**
**Tokyo (JP)**

(72) Inventors:
 • **Hayashi, Ryuzo**
  **Osaka 577 (JP)**
 • **Hashizume, Yoshio**
  **Akashi-shi, Hyogo-ken, 673 (JP)**
 • **Kariyone, Akio**
  **Kyoto, 603 (JP)**

(74) Representative: **Beetz & Partner Patentanwälte**
**Steinsdorfstrasse 10**
**80538 München (DE)**

(56) References cited:
 **EP-A- 0 335 167**

 • **ANALYTICA CHIMICA ACTA vol. 230, no. 1, 1**
  **March 1990, AMSTERDAM NL pages 75 - 82 T.**
  **IKEDA ET AL. 'Amperometric response to**
  **reducing carbohydrates of an enzyme electrode**
  **based on oligosaccharide dehydrogenase.**
  **Detection of lactose and alpha-amylase.'**

## Description

This invention relates to the technique of determining the average molecular weight (Ma), average chain length (Dp) or dextrose equivalent (DE) value of starch and/or its related carbohydrates, including polysaccharides, and oligosaccharides containing glucose units, and of other oligosaccharides and polysaccharides containing or consisting of aldose sugar monomer units, with convenience and high accuracy, wherein the related carbohydrates particularly comprise hydrolysates from starch, including linear maltooligosaccharides like maltose, maltotriose, maltotetraose, maltopentaose, maltohexaose, maltoheptaose, and derivatives of starch or hydrolysates thereof, for example, ether derivatives, cationic derivatives, and carboxyl derivatives. Objects of measurement are, for example, sweetening products, corn syrup, food additives, and other industrial materials.

In the following, reference is made to starch and related carbohydrates, however the invention is not limited to the analysis of these products.

Starch and related carbohydrates (hereinafter the term "starch" may include such related matter) have been widely used as materials or additives for manufacturing food, paper, and various other industrial products. In such industrial applications of starch, it is important to know the molecular weight (MW) of the starch to be applied as a factor indicating its properties. For example, the molecular weight (MW) affects the viscosity of a starch solution, the hygroscopic property, or the sweet taste in the case of corn syrup. Conventional methods for determining the molecular weight (MW) have been: Measurement by intrinsic viscosity, light scattering, osmotic pressure, high speed liquid chromatography or quantitative determination of reducing terminal groups contained in starch. Of them, the intrinsic viscosity method and the light scattering method include complicate processes for preparing the sample for measurement and take a long time for a measurement. The osmotic pressure method is likely to be influenced by the concentration of salts, and it is necessary to be careful in preparing the sample for measurement. Therefore these methods have been rarely employed except in the basic pre-industrial research stage. The high speed liquid chromatography is versatile in the point of determining the average molecular weight (MW) as well as the molecular weight distribution, but this method requires a standard sample for the molecular weight (MW), a complicate process for preparing the sample to be measured, a relatively expensive apparatus, and a time consuming work for measurement. Accordingly, this method is also unsuited for industrial applications.

Consequently, the most conveniently applicable approach is the quantitative measurement of reducing terminal groups, which includes the determination of the total amounts of reducing terminal groups and glucose units contained in a starch sample, and then the ratio of these two amounts is formed representing the molecular weight (MW). In the conventional art of determining the whole glucose units, determination of gross carbohydrate by the phenol and sulfuric acid method has been used, wherein strong acidic and alkaline chemicals are involved, and heating is occasionally necessary. These factors posed problems regarding the disposal of waste chemical liquids and also the hazardous operation. In view of these problems, for the sake of convenience, in the case where an outstanding starch solution does not contain any other chemicals than carbohydrates, the dry weight thereof was used as a substitute for the gross sugar amount. Thus the DE value as an index of the molecular weight is derived from forming the ratio between the dry weight as noted above and the quantitative amount of reducing terminal groups. However, actually starch is hygroscopic which makes it difficult to determine the correct dry weight.

Some of the methods for determining the reducing terminal groups are to use organic agents which react with the reducing terminal groups, and to use the oxidation and reduction of ions such as Fe ions and Cu ions. Of them above all, the oxidation and reduction of Cu ion has been used as the basis of measurement. That is, when divalent copper and a reducing sugar are heated in an alkaline solution, the copper is reduced to monovalent copper which may be quantitatively determined by colorimetry, spectrophotometry, or titration. However, this method has the disadvantages of heating a mass of alkaline solution, hazardous operation and of producing a mass of waste liquid including copper. In addition, while the reducing sugar reduces $Cu^{2+}$ ions to $Cu^{1+}$ ions, oxygen of air dissolving in the reacting solution, may cause a reoxidation of the monovalent Cu ions. Thus, errors are produced. For preventing such errors, quick operation and sufficient skill are necessary, but actually the human error plays a significant role.

For the reasons explained above it becomes clear, that means for determining the average molecular weight (MW), the average chain length (hereinafter often referred to as molecular weight (MW) or equivalent) or the DE value of starch are of industrially large significance, and that there was a need for a method for determining these properties with convenience and accuracy.

EP 335 167 A discloses an apparatus for measuring malto-oligosaccharides comprising an immobilized enzyme membrane containing glucoamylase and glucose oxidase wherein the diffusion rate of the substrate sample into the enzyme layer and the substrate consumption rate are balanced by defining a specific rate ratio so that the rate of product formation in the enzyme layer is proportional to the substrate concentration in the sample solution. This document does not deal with the determination of average molecular weight, average chain length oder dextrose equivalent of aldose-based oligo- or polysaccharides.

As noted above, the term "starch" will be used to represent not only starch, but also its related carbohydrates, including hydrolysates from starch, derivatives of starch or hydrolysates, and other oligosaccharides and polysaccharides comprising glucose units, for sake of simplicity of the description.

It is the object of the invention to provide a method for determining the molecular weight (MW) or a molecular weight equivalent of starch with convenience and accuracy, wherein the use of metal ions and strong acidic or strong alkaline chemicals is avoided.

This object is achieved in accordance with claim 1. The dependent claims relate to preferred embodiments.

The method of the present invention for determining the average molecular weight, Ma, the average chain length, Dp, and/or the dextrose equivalent value, DE, of oligosaccharides or polysaccharides containing or consisting of aldose sugar monomer units, particularly glucose or galactose units, and/or related carbohydrates comprises the following steps:

(A) Quantitative determination of the gross aldose amount (Gt) of a hydrolyzed sample by means of an immobilized enzyme electrode or a combination of an immobilized enzyme column and an electrode sensitive to an enzyme reaction product,

(B) Reduction of reducing terminal groups of a sample and quantitative determination of the aldose amount (Gr) of the hydrolyzed sample by means of an immobilized enzyme electrode or a combination of an immobilized enzyme column and an electrode sensitive to an enzyme reaction product, and

(C) Calculation of the average molecular weight Ma, the average chain length Dp and/or the DE value from the gross aldose amount (Gt) and the aldose amount (Gr) in accordance with predetermined relations.

In the particular case of this method for the analysis of oligosaccharides or polysaccharides containing or consisting of glucose units, and particularly starch and/or related carbohydrates, the method of the present invention comprises the following steps:

(A) Quantitative determination of the gross glucose amount (Gt) of a hydrolyzed sample by means of an immobilized enzyme electrode or a combination of an immobilized enzyme column and an electrode sensitive to an enzyme reaction product,

(B) Reduction of reducing terminal groups of a sample and quantitative determination of the glucose amount (Gr) of the hydrolyzed sample by means of an immobilized enzyme electrode or a combination of an immobilized enzyme column and an electrode sensitive to an enzyme reaction product, and

(C) Calculation of the average molecular weight Ma, the average chain length Dp and/or the DE value from the gross glucose amount (Gt) and the glucose amount (Gr) in accordance with predetermined relations.

Particularly, in determining the molecular weight (MW) or an equivalent thereof, this invention employs an enzyme electrode or a combination of an immobilized enzyme column and an electrode which provides specificity and achievement of high speed measurement, thereby overcoming the conventional difficulties in convenience, accuracy and promptness.

More particularly, this invention employs an immobilized enzyme electrode, or a combination of an immobilized enzyme column and an electrode, to determine the gross glucose amount contained in hydrolysates from starch, referred to as step (i), and to determine the glucose amount contained in hydrolysates from starch of which reducing terminal groups have been beforehand reduced, referred to as step (ii). As step (iii), this invention includes the calculation of the average molecular weight Ma, the average chain length Dp, or of the DE value, based on measurements in the steps (i) and (ii).

For reducing the reducing terminal groups of starch, the use of a compound of borohydride is preferable. In hydrolyzing the starge, prior or subsequent to the reduction, the use of amylase or the combined use of glucosidase and amylase is preferable.

The method of this invention is preferably carried out with a device provided with an immobilized enzyme electrode or a combination of an immobilized enzyme column and an electrode (a) for achieving a measurement of the free glucose amount, and (b) for achieving hydrolysis of a starch sample to determine the gross glucose amount, and (c) for achieving hydrolysis of the starch sample of which reducing terminals have been beforehand reduced, to determine the glucose amount subsequent to the reduction, and provided with means (d) for calculating the average molecular weight (MW), the average chain length Dp, and the DE value, based on the measurements of the free glucose amount, and the glucose amounts with and without reduction.

The method of this invention is preferably carried out with an apparatus which comprises

- measuring means for the quantitative electrochemical determination of the aldose and particularly the glucose amount (Gf; Gt; Gr) of a sample, comprising an immobilized enzyme electrode or a combination of an immobilized enzyme column and an electrode sensitive to an enzyme reaction product, and

- determination means for determining the average molecular weight Ma , the average chain length Dp and/or the DE value from the aldose and particularly the glucose amount (Gf; Gt; Gr) or respective data measured by the measuring means in accordance with predetermined relations.

According to a prefered embodiment of the present method, the determination means of the apparatus used are designed such as to calculate the average molecular weight Ma, the average chain length Dp and/or the dextrose equivalent DE value according to the following equations [2], [3] or [4]:

$$Dp = 2 \cdot Gt/(Gt - Gr) \qquad [2]$$

$$Ma = 162 \cdot Dp \qquad [3]$$

$$DE = 100(Gt - Gr)180/2 \cdot 162 \cdot Gt \qquad [4].$$

In accordance with another preferred embodiment relating to samples containing free glucose, the free glucose amount Gf of the sample, the gross glucose amount prior to the reduction Gt, and the glucose amount subsequent to the reduction Gr are determined. Preferably, the apparatus used includes means for storing these data, from which the average molecular weight Ma, the average chain length Dp, and/or the DE value are calculated with suitable means particularly according to the equations [6], [7], [9], or [10], respectively:

$$Dp = 2 \cdot Gt/(Gt - Gr + Gf) \qquad [6]$$

$$Ma = 18(19 \cdot Gt + Gf - Gr)/(Gt - Gr + Gf) \qquad [7]$$

$$DE = 1000(Gt - Gr + Gf)/(19 \cdot Gt - Gr + Gf) \qquad [9]$$

$$DE = 1000/(9 \cdot Dp + 1) \qquad [10].$$

In accordance with further preferred embodiments, the method of the present invention is characterized in that the apparatus used has at least one of the following features:

- The apparatus is of batch type, flow type or stopped-flow type;

- the measuring means are provided in a thermostat ;

- the measuring means comprise an immobilized enzyme electrode , a reference electrode , a counter electrode and a potentiostat ;
or comprise an immobilized enzyme column , a reference electrode , a counter electrode , a $H_2O_2$ electrode or an $O_2$ electrode and a potentiostat ;

- the measuring means comprise a flow pipe provided upstream and comprising an injector at its intake end;

- the immobilized enzyme electrode or the immobilized enzyme column comprise immobilized galactose oxidase, and glucose oxidase or pyranose oxidase;

- the electrode is an $H_2O_2$ or an $O_2$ electrode;

- the electrodes , the reference electrode and the counter electrode are provided in a measurement cell ;

- the determination means comprise an A/D converter for A/D converting the data from the potentiostat to be input into the computer or microprocessor system ,

- the computer or microprocessor system comprises means for storing, transferring output and/or displaying data and/or results, particularly Ma, Dp and/or DE values;

- the determination means comprise a printer for printing data and/or results, particularly Ma, Dp and/or DE values;

- the computer comprises a keyboard for data input and operation;

- the apparatus further comprises means for hydrolyzing the samples before the aldose and particularly glucose measurement;

- the apparatus further comprises means for the reduction of reducing terminal groups of the samples;

- the apparatus is automatized in such a manner that the values of Gt and Gr, and eventually also of Gf, are automatically determined from one or more samples, and the results (Ma, Dp, DE value) are automatically calculated and output therefor.

In the following, the invention will be explained with reference to preferred embodiments and to the drawings wherein:

Fig. 1    is a schematic drawing of a flow type apparatus which is employed in the examples 1 and 2 as will be apparent later.

Fig. 2    is a flow chart showing the sequence of operations in the analysis.

Fig. 3    is a schematic drawing of a flow-type apparatus including a combination of an immobilized enzyme column and an electrode, employed in example 3.

A.

At first, the process of hydrolyzing starch to determine the gross amount of glucose will be described. This invention uses a device including an enzyme electrode or a combination of an immobilized enzyme column and an electrode for measuring glucose.

As for hydrolysis, acidic hydrolysis may be applied, but this method requires heating at a high temperature of about 120 °C for several hours; thereby this method is prone to cause decomposition of glucose and thus a good recovery rate is difficult to obtain. Therefore, enzymatic hydrolysis is preferable.

In the enzymatic hydrolysis of starch, the use of amylase or the combined use of glucosidase and amylase is preferable.

As for species of amylases, α-amylase (EC 3. 2. 1. 1) or glucoamylase (EC. 3. 2. 1. 3) are preferable. Of them, glucoamylase is especially preferable because it works with a large decomposition rate, and the enzyme itself is stable. However, glucoamylase lowers its activity with shorther starch chain length. For compensation, α-glucosidase (EC 3. 2. 1. 20) having good activity to oligo sugars is preferably added. Also, as for samples which have high molecular weight and are difficult to dissolve, the use of both α-amylase and glucoamylase is preferred because it prevents the retrogradation of starch in hydrolysis.

The enzyme electrode for measuring glucose is not particular or restricted but it may be defined by comprising an oxygen electrode or a hydrogen peroxide electrode provided in close vicinity to glucose oxidase (EC 1. 1. 3. 4) or pyranose oxidase (EC. 1. 1. 3. 10) in immobilized form.

The apparatus incorporating the enzyme electrode may be a batch system or a flow system or another arrangement. Of them, the flow type apparatus incorporating the enzyme electrode with detection of hydrogen peroxide is preferable in view of high speed and accuracy. Also preferable is the flow type measurement apparatus, upstream thereof a column type reactor including immobilized glucose oxidase or pyranose oxidase being arranged and downstream thereof the oxygen electrode or hydrogen peroxide electrode being arranged.

B.

Next, the description is given of the process for determining glucose produced by hydrolysis subsequent to reduction of the reducing terminal groups of starch. In this invention, objectives for measurement to determine gross glucose are limited to carbohydrates, and therefore there is no need to be cautious in view of a possible interaction of oxygen as compared to the case of determining the reducing group by means of Cu ions. For the purpose of finding how much the carbohydrate has changed, a measurement of a decrease of the carbohydrate amount is acceptable by oxidizing the reducing terminal groups, however, the reduction method is preferable to avoid waste liquor disposal problems. Then, reduction of the reducing terminal groups of carbohydrates may be carried out by means of a catalyst and hydrogen gas or by adding a hydride compound. Of them, the use of a borohydride compound is preferable because the final decomposition product thereof is boric acid, and thereby the problem of waste liquor is solved. It has been found that boric acid of practical concentrations at which this invention is carried out does not have any unfavorable effect to an enzyme electrode, and that the reduction rate is fast to effect prompt processing. Water soluble borohydride compounds such as sodium borohydride and potassium borohydride may be used.

In carrying out the reduction with use of these compounds, it is possible to put a borohydride compound in solid form into a water solution containing the starch sample, or to dissolve the borohydride compound in water or a lower alcohol beforehand so that a liquid form of the borohydride compound may be put into the solution containing the starch sample. In the latter case, decomposition of borohydride compound takes place gradually with time; accordingly, it is recommendable to prepare the solution just before use. Decomposition of borohydride compounds also occurs under acidic conditions, which may cause incomplete reduction. Therefore, it is preferable to keep the pH value of the solution of the starch sample in the neutral or alkaline range. The amount of the borohydride compound to be added should be equal to or more than molar balance to possibly existing amount of reducing sugar. However, should the charged mass be much in excess, the presence of the remnant borohydride compound may possibly interfere with the subsequent analysis, and therefore, an excess from 2 to 10 times may be preferable. This reducing reaction proceeds at room temperature, though heating up to 30 to 100 °C is desirable to promote the reaction. Normally the reaction at about 50 °C takes about 5 min for completion.

In turn, the reduced product thus obtained is subjected to hydrolysis. At this stage, if an excess of borohydride compound should be present, glucose generated by hydrolysis would be reduced further, which renders the measurement to be unreasonably lower. In order to avoid this possible trouble, the solution is preferably made acidic beforehand to decompose the existing borohydride compound, for which purpose titration with a diluted hydrochloric acid, 1N HCl solution, for example, is recommended.

In the process of hydrolyzing a reduced starch or related carbohydrates, use of enzyme-based chemical means is preferable likewise in the step of determining the gross glucose amount. It is to be noted here that at the reduced state, reduced starch or carbohydrates take maltitol structure. It is impossible for both $\alpha$-amylase and glucoamylase to cleave the $\alpha$-1,4 linkage present in maltitol molecules. Therefore, the mole number of reducing terminal glucose groups in starch or related carbohydrates is twice the difference (Gt - Gr) of the glucose amount Gt obtained after amylase hydrolysis and the amount of glucose Gr obtained after hydrolysis of the reduced product. It is like raising the sensitivity twice in the measurement.

In cases where it is necessary to directly determine the amount of reducing terminal groups which is derived by subtraction of the glucose amount Gr from the gross glucose amount Gt, the combined use of $\alpha$-glucosidase is recommended. Particularly, $\alpha$-glucosidase produced by yeast has relatively lower specificity and catalytic activity for hydrolysis of maltitol compounds.

C.

In the following, the process for calculating the average molecular weight (MW), the average chain length Dp or the DE value based on the gross glucose amount Gt and the glucose amount Gr will be described. These results may be derived from measurements in the steps noted above.

The following example refers to the case where glucoamylase is used for the hydrolysis. It is assumed that Gt indicates the number of moles of gross glucose prior to the reduction, and that Gr indicates the number of moles of glucose produced by the hydrolysis subsequent to the reduction. First, the number of moles of reducing terminal groups R is given, with consideration that maltitols are not hydrolyzed through glucoamylase subsequent to the reduction, as follows:

$$R = (Gt - Gr)/2 \hspace{3cm} [1].$$

The average chain length Dp is determined by division of the gross glucose mole number Gt by R as follows:

$$Dp = 2 \cdot Gt/(Gt - Gr) \hspace{3cm} [2].$$

Based on the above and on the fact that a glucose unit in condensed state (elimination of a water molecule) has the average molecular weight (MW) 162, the average molecular weight Ma of a starch or carbohydrate sample is given as follows:

$$Ma = 162 \cdot Dp \hspace{3cm} [3].$$

The DE value is a value which is derived basically by multiplication 100 times the ratio of the glucose equivalent amount of a reducing sugar in relation to the total solid amount, which leads to the following expression:

$$DE = 100(Gt - Gr)180/2 \cdot 162 \cdot Gt \qquad [4].$$

These expressions [1] to [4] are correct if no free glucose is contained.

Accordingly, it is to be noted here that some correction factor should be taken into account if free glucose is contained in a considerable amount in a sample, for example, in partial hydrolysates of starch.

As for free glucose, a starch or carbohydrate sample is diluted to a solution, which is analyzed with use of an enzymatic electrode for measuring glucose, whereby the amount of free glucose Gf is determined. Then, the molar number of reducing terminal groups is given like the expression 1 as below:

$$R = ((Gt - Gf) - Gr)/2 + Gf.$$

The above leads to:

$$R = (Gt - Gr + Gf )/2 \qquad [5].$$

The average chain length Dp is modified as below:

$$Dp = 2 \cdot Gt/(Gt - Gr + Gf) \qquad [6].$$

The accurate average molecular weight Ma is given as below:

$$Ma = (342(R - Gf) + 162 \cdot Gr + 180 \cdot Gf)/R.$$

The above leads to:

$$Ma = 18(19 \cdot Gt + Gf - Gr)/Gt - Gr + Gf) \qquad [7].$$

Likeweise, the amounts of all solid components Ts may be determined:

$$Ts = 180 \cdot Gf + 342(R - Gf) + 162 \cdot Gr.$$

The above leads to:

$$Ts = 9(19 \cdot Gt - Gr + Gf) \qquad [8].$$

In turn, the DE value is modified as below:

$$DE = (180 \cdot R/Ts) \times 100.$$

The above leads to:

$$DE = 1000(Gt - Gr + Gf)/(19 \cdot Gt - Gr + Gf) \qquad [9].$$

The above may be represented as follows:

$$DE = 1000/(9 \cdot Dp + 1) \qquad [10].$$

The DE value is practically acceptable when characterization or quality control of carbohydrates is intended, but for research purposes, the average chain length Dp is often used, wherein Dp multiplied by the molecular weight of the structural unit or monomer gives the average molecular weight of the polysaccharide. As has been known, many characteristics of high polymers are related to the average molecular weight, and therefore, determination of Dp is significant. Equation 10 is particularly suitable for interpreting DE in the research practice.

As is understood from the calculation sequences noted above, the method and apparatus of the present invention are not affected by the coexistence of reducing sugars such as galactose, fructose, and others, and are not influenced by the presence of any salt derived from other materials than starch or carbohydrates, because the enzyme decomposes only carbohydrates mainly consisting of glucose. This selectivity is particularly advantageous. In addition, glucoamylase does not cleave linkages except $\alpha$-1,4 and $\alpha$-1,6-glucosides, and thus, the presence of sucrose and the like compounds does not interfere with the measurements. Needless to mention, that in cases where a sample does not include compounds other than starch and related carbohydrates, it is possible to calculate the ratio of the amount of reducing terminal groups to the absolute dry weight.

The concept as described herein may be also utilized for the analysis of polymerization degrees of polysaccharides consisting of glucose such as pullulan, dextran, for example, by suitable adaptation of the enzymatic reactions to be applied to the hydrolysis. In the case of pullulan, pullulanase (EC 3. 2. 1. 41) which will break $\alpha$-1,6 linkages, and glucoamylase which will break $\alpha$-1,4 linkages, are recommended to be jointly used, whereby a degradation down to the glucose unit is feasible. Therefore, if (i) a sample is subjected to complete degradation to glucose, and its glucose concentration is determined, and then (ii) similar operations including the same degradation subsequent to reduction of reducing terminal groups are carried out, similar results to those found on starch are obtained. Glucoamylase will also break α-1,6 linkages, however, its rate is slow as compared to the activity on α-1,4 linkages, and therefore the joint use with pullulanase is practical.

In the case of dextran, dextranase (EC 3. 2. 11) will enable degradation down to glucose, and therefore a similar analysis is feasible. Dextran and pullulan are popular polysaccharides produced by microorganisms.

Further, the invention may be applied to carry out analyses of other natural and synthetic polysaccharides made up of other monomers, wherein a suitable enzyme electrode or immobilized enzyme column are used. Accordingly, it is also possible to measure these other polysaccharides with use of a combination of the respective polysaccharide decomposing enzyme and an electrode sensitive to a suitable product. Taking galactan contained in plants as an example, the amount of DE value equivalent (wherein the DE value itself is originally defined with regard to starch and the related carbohydrates) of galactan may be calculated by combining a hydrolase which has an activity to cut the galactoside linkage and an immobilized galactose oxidase electrode.

In the same way, the analysis of cellooligosaccharides which have mainly β-1,4 linkages may be carried out with hydrolysis by employing a highly activated species of cellulase which will hydrolyze cellulose to glucose. In this case, the glucose electrode is employed.

D.

Although the calculations as noted above may be carried out manually, a combined equipment of a suitable data processing device, particularly a computer system, with the apparatus for measurements will facilitate a convenient, quick and easy performance of analytical work.

The apparatus as used in the present invention for the analysis of oligosaccharides or polysaccharides containing aldose and particularly glucose units, and particularly of starch and/or related carbohydrates, which is particularly suited for carrying out the method as defined above, comprises

- measuring means for the quantitative electrochemical determination of the aldose and particularly the glucose amount (Gf; Gt; Gr) of a sample, comprising an immobilized enzyme electrode or a combination of an immobilized enzyme column and an electrode sensitive to an enzyme reaction product, and

- determination means for determining the average molecular weight (Ma), the average chain length (Dp) and/or the DE value from the aldose and particularly the glucose amount (Gf; Gt; Gr) or respective data measured by the measuring means in accordance with predetermined relations.

Preferably, in the method of this invention, an apparatus is used employing an immobilized enzyme electrode or a combination of an immobilized enzyme column and an electrode (1) for achieving the measurement of the free glucose amount contained in starch, (2) for the determination of the glucose amount derived from hydrolysis of the starch, and (3) for the determination of the glucose amount of the starch subsequent to reduction. This apparatus further has means for calculating the molecular weight (MW) or an equivalent therefor based on the free glucose amount Gf and the glucose amounts Gt and Gr with and without reduction, respectively. The apparatus preferably has means for storing these data, from which the average molecular weight Ma, the average chain length Dp, and the DE value are calculated by means of the equations [7], [6], [9], and [10] as indicated above.

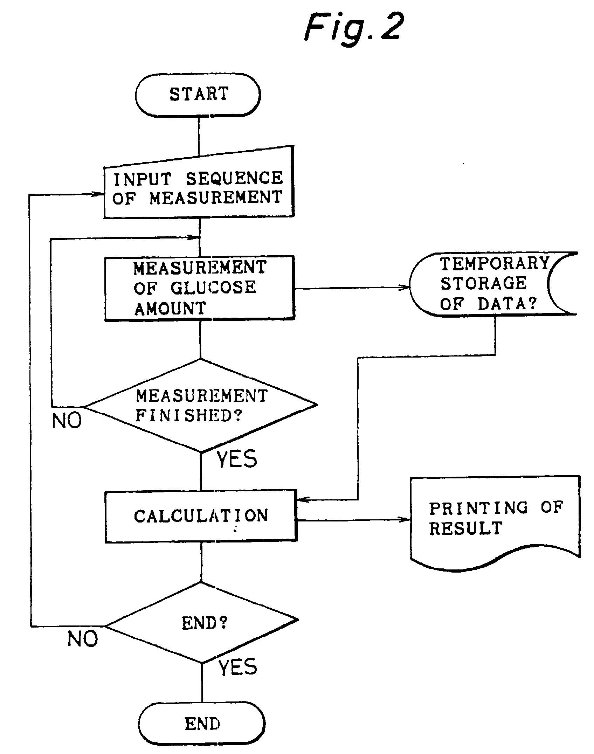

Referring briefly to the mechanism for the determinations and calculations, the detected voltages generated at the terminals of the potentiostat (the device comprising a measuring electrode, a counter electrode and a reference electrode in the potentiostat measuring system) are stored as data subsequent to A/D conversion. The calculations according to the expressions noted above may be performed independently from the measurements, or the start of the calculations may coincide with the completion of the measurements, following previous instructions as to what sequence should be kept. The flow chart referring to the sequence of measurements and calculations is shown in Fig. 2 as will apparent later. Therein, if e.g. the measurement items relate to different dilution factors, suitable corrections will be applied.

These sequential processings may be carried out by a single board computer. Alternatively, the data may be transferred to a personal computer wherein the data are processed by programs, and wherein data may be stored in suitable memories, e.g. magnetic memory media.

**Example 1**

Apparatus for Measurement

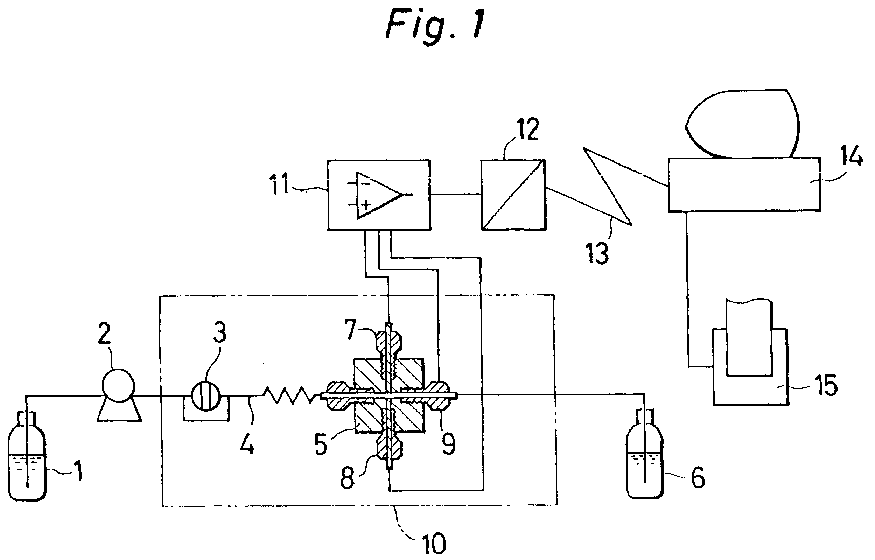

Measurements of glucose concentrations were carried out with the flow type apparatus as shown in Fig. 1, wherein a pump 2 for high speed liquid chromatography was used to feed a 100 mM sodium phosphate buffer solution 1 (pH 6.0) at a rate of 1.0 ml/min. A sample to be analyzed was injected with use of an injector 3. The injected sample was designed to flow, through a pipe 4 made of fluorocarbon resin having an inner diameter of 0.5 mm and a length of 1.0 m, to a measurement cell 5 which was connected to an injector, and then to a waste bottle 6. The measurement cell 5 was provided with an enzyme electrode 7 for measurement of glucose on which glucose oxidase was immobilized with bovine serum albumin, an Ag/AgCl reference electrode 8, and a counter electrode 9. The cell was mounted in a thermostat 10 to be kept at a temperature of $37 \pm 0.2$ °C.

With use of a potentiostat 11, a voltage of + 0.6 V was impressed to the enzyme electrode with reference to the reference electrode, and thereby, the output current values from the enzyme electrode were transferred, after digitizing by an A/D converter 12, to a personal computer 14 through a cable 13. Recording and analysis were carried out in the computer 14, and the results were printed out by a printer 15.

Measurement of Free Glucose

A solution of 1.00 ml was taken into a test tube from each of the following samples G1 to G7, to which a 4.00 ml of distilled water were added. This diluted samples were fed to the glucose sensor apparatus, and the measured glucose concentrations were multiplied 5 times, which was assumed to be the free glucose amount Gf of each of samples G1 to G7 as below:

50.0 mM glucose G1
25.0 mM maltose G2
16.7 mM maltotriose G3
12.5 mM maltotetraose G4
10 mM maltopentaose G5
8.33 mM maltohexaose G6
7.14 mM maltoheptaose G7.

Measurement of Gross Glucose Amount

Each solution G1 through G7 of 1.00 ml was taken into a test tube, to which 5 µl of glucoamylase (derived from <u>Aspergillus niger,</u> purchased from SIGMA Co.) and 3995 µl of 1000 mM citric acid buffer solution (pH = 5.0) were added, and the resulting solution was kept at 37 °C for 1 hour. The glucoamylase liberated glucose step by step by cutting the glucose chain at its non-reducing terminal side. The sample thus prepared was fed into the glucose sensor apparatus, and the measured glucose concentration was multiplied 5 times, which was assumed to be the gross glucose amount Gt for each of samples G1 to G7.

Measurement subsequent to Reduction by Sodium Borohydride

Each of the solutions G1 to G7 of 1.00 ml was taken into a test tube, whereinto 100 µl of 0.1 g/ml sodium borohydride solution were added. The mixture was then kept in a 37 °C water bath for 15 min. After completion of the reduction, unreacted sodium borohydride was decomposed by addition of 500 µl of 1 N hydrochloric acid. Then 5 µl glucoamylase and 100 mM citric acid buffer solution (pH = 5.0) were added to become 5.00 ml solution, with which glucose was obtained through reaction at 37 °C for 30 min. This solution was injected into the glucose sensor apparatus, and the resulting glucose concentration was multiplied 5 times, which was assumed to be the glucose amount Gr subsequent to reduction obtained with solutions G1 to G7.

Determination of Average Chain Length and DE Value

The respective data resulting from the analyses were inserted into equation 6 to calculate the average chain length, and by using the data in equation 9 . DE values were calculated, as shown in Table 1.

Theoretical DE values calculated from the molecular formulae of G1 to G7 and the experimentally determined values were found to agree with each other with a correlation coefficient of 0.999 or more. As for the average chain length, the experimental results agree with the theoretical values with a correlation coefficient of 0.999 or more.

TABLE 1

|  | Theor. CL * | Theor. DE | Exp. aCL ** | Exp. DE |
|---|---|---|---|---|
| G1 | 1 | 100 | 1.00 | 100 |
| G2 | 2 | 52.6 | 2.00 | 52.6 |
| G3 | 3 | 35.7 | 2.95 | 36.3 |
| G4 | 4 | 27.0 | 3.91 | 27.6 |
| G5 | 5 | 21.7 | 4.88 | 22.3 |
| G6 | 6 | 18.2 | 6.09 | 17.9 |
| G7 | 7 | 15.6 | 6.90 | 15.8 |

\* CL = chain length

\*\* aCL = average chain length.

## Example 2

Five samples were prepared each having a DE value as below, measured by the Lane Eynon method which is representative of methods including oxidation and reduction of copper. The DE values were 30.0, 32.5, 35.0 and 40.0. These samples were found by means of liquid chromatography to contain glucose polymers having various chain lengths. The samples were diluted in water to solutions of 1 % (wt/vol) and then subjected to experiments and sequential calculations similar to example 1. The results show good agreement with data obtained by Lane Eynon method as shown in Table 2.

TABLE 2

| Measurements by Lane Eynon Method | Measurements in Example 2 |
|---|---|
| 30.0 | 29.8 |
| 32.5 | 32.5 |
| 35.0 | 35.1 |
| 37.5 | 37.4 |
| 40.0 | 40.1 |

The Lane Eynon method is based on the analysis by reduction of Cu ions contained in an alkaline solution with a reducing sugar solution added, and following determination of remaining Cu ions by titration.

## Example 3

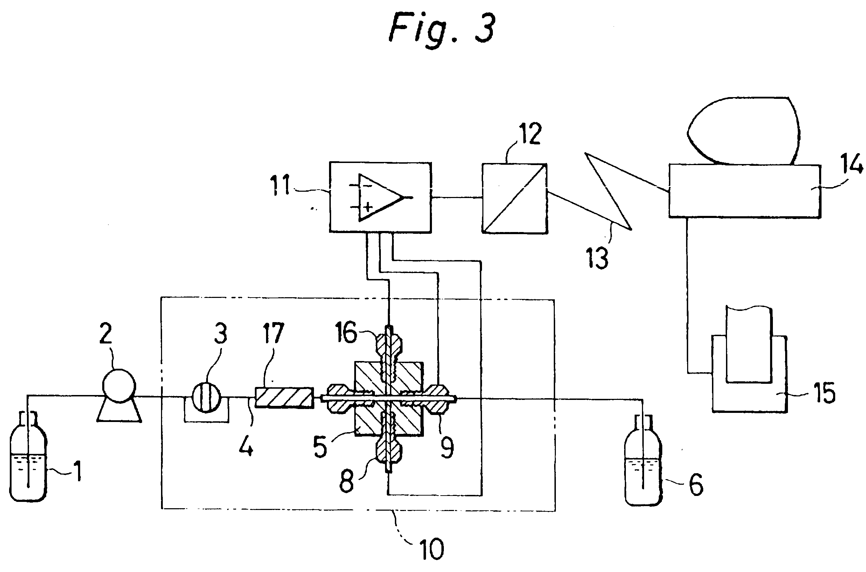

The apparatus used for measuring glucose by means of a combination of an immobilized enzyme column and an electrode is shown in Fig. 3. First, the preparation of the immobilized enzyme column 17 in Fig. 3 will be explained. 150 mg of fire brick particles (sieved into 30 to 60 meshes) were well dried, and thereon 1 ml of γ-aminopropyltriethoxy silane in a 10 % anhydrous toluene solution was added and allowed to stand for 1 hour. The silane coupling agent was washed out well with toluene and ethanol, and then the product was dried at 120 °C for 2 hours. After cooling in the air, 0.5 ml of 5 % glutaraldehyde solution was added, and the mixture was allowed to stand at room temperature for 1 hour. This carrier was then washed well with water. It was finally washed with 100 mM sodium phosphate buffer solution (pH 7) whereupon the buffer solution was removed as much as possible. The amino silanated carrier thus obtained was loaded with an enzyme solution containing 10 mg of glucose oxidase (purchased from SIGMA) in a sodium phosphate buffer solution (pH 7) and allowed to stand at a room temperature for 1 hour. It was then washed with the buffer solution. The immobilized enzyme carrier thus obtained was filled into a tube made of fluorocarbon resin having an outer diameter of 3 mm, an inner diameter of 2 mm and a length of 10 cm.

The immobilized enzyme column 17 was fitted in the apparatus as shown in Fig. 3. The features of the apparatus were practically the same as in that of example 1 (Fig. 1), wherein an electrode 16 for measuring hydrogen peroxide 16 was used in place of the glucose measuring electrode 7 of Fig. 1.

Analyses were carried out on the same samples as in example 2. The obtained results show good agreement with the results obtained by the Lane Eynon method as shown below in Table 3.

TABLE 3

| Measurements by Eynon Lane Method | Measurements in Example 3 |
|---|---|
| 30.0 | 30.1 |
| 32.5 | 32.6 |
| 35.0 | 34.9 |
| 37.5 | 37.4 |
| 40.0 | 40.0 |

## Claims

1. A method for determining the average molecular weight (Ma), the average chain length (Dp) and/or the dextrose equivalent (DE) value of oligosaccharides or polysaccharides containing or consisting of aldose sugar monomer units, particularly glucose or galactose units, and/or related carbohydrates,
comprising the following steps:

(A) Quantitative determination of the gross aldose amount (Gt) of a hydrolyzed sample by means of an immobilized enzyme electrode or a combination of an immobilized enzyme column and an electrode sensitive to an enzyme reaction product,

(B) Reduction of reducing terminal groups of a sample and quantitative determination of the aldose amount (Gr) of the hydrolyzed sample by means of an immobilized enzyme electrode or a combination of an immobilized enzyme column and an electrode sensitive to an enzyme reaction product,
and

(C) Calculation of the average molecular weight (Ma), the average chain length (Dp) and/or the dextrose equivalent (DE) value from the gross aldose amount (Gt) and the aldose amount (Gr) in accordance with predetermined relations.

2. The method according to claim 1 for the analysis of oligosaccharides or polysaccharides containing or consisting of glucose units, and particularly starch and/or related carbohydrates,
comprising the following steps:

(A) Quantitative determination of the gross glucose amount (Gt) of a hydrolyzed sample by means of a immobilized enzyme electrode or a combination of an immobilized enzyme column and an electrode sensitive to an enzyme reaction product,

(B) Reduction of reducing terminal groups of a sample and quantitative determination of the glucose amount (Gr) of the hydrolyzed sample by means of an immobilized enzyme electrode or a combination of an immobilized enzyme column and an electrode sensitive to an enzyme reaction product,
and

(C) Calculation of the average molecular weight (Ma) the average chain length (Dp) and/or the dextrose equivalent (DE) value from the gross glucose amount (Gt) and the glucose amount (Gr) in accordance with predetermined relations.

3. The method according to claims 1 and 2, wherein a borohydride, preferably sodium and/or potassium borohydride, is used in step B for the reduction of reducing terminal groups of the sample.

4. The method according to claims 1 to 3, wherein an amylase, preferably an α-amylase, and/or a glucoamylase and/or a glucosidase, preferably an α-glucosidase, are used for the hydrolysis of the sample prior to or after the reduction of reducing terminal groups.

5. The method according to claims 2 to 4, characterized in that, for samples wherein no free glucose is contained and for the case of using glucoamylase for the hydrolysis, the average chain length (Dp), the average molecular weight (Ma) or the dextrose equivalent (DE) value are calculated according to the following equations [2], [3] or [4]:

$$Dp = 2 \cdot Gt/(Gt - Gr) \qquad [2]$$

$$Ma = 162 \cdot Dp \qquad [3]$$

$$DE = 100(Gt - Gr)180/2 \cdot 162 \cdot Gt \qquad [4].$$

6. The method according to claims 1 to 4, characterized by quantitative determination of the free aldose or glucose amount (Gf), respectively, comprised in the sample before step A or independently, and calculation of the average molecular weight (Ma), the average chain length (Dp) and/or the dextrose equivalent (DE) value from the free aldose or glucose amount (Gf), the gross aldose or glucose amount (Gt) and the aldose or glucose amount (Gr).

7. The method according to claims 1 to 4 and 6, characterized in that, for the case of glucose-based oligo- and polysaccharides, in step C, the average molecular weight (Ma), the average chain length (Dp) or the dextrose equivalent (DE) value are calculated according to the following equations [6], [7] , [9] or [10]:

$$Dp = 2 \cdot Gt/(Gt - Gr + Gf) \qquad [6]$$

$$Ma = 18(19 \cdot Gt + Gf - Gr)/(Gt - Gr + Gf) \qquad [7]$$

$$DE = 1000(Gt - Gr + Gf)/(19 \cdot Gt - Gr + Gf) \qquad [9]$$

$$DE = 1000/(9 \cdot Dp + 1) \qquad [10].$$

8. The method according to claims 1 to 7, characterized in that the gross aldose or glucose amount (Gt), respectively, and/or the aldose or glucose amount (Gr) are determined in steps A and/or B after hydrolysis of the respective samples with use of an immobilized enzyme electrode for detecting the aldose, particularly glucose, comprising an immobilized aldose oxidase and particularly immobilized glucose oxidase or pyranose oxidase, and/or with use of an immobilized enzyme column comprising an immobilized aldose oxidase and particularly immobilized glucose oxidase or pyranose oxidase, and of a hydrogen peroxide electrode or an oxygen electrode.

9. The method according to claims 1 to 8 carried out with an apparatus comprising

   - measuring means (7; 16, 17) for the quantitative electrochemical determination of the aldose and particularly the glucose amount (Gf; Gt; Gr) of a sample, comprising an immobilized enzyme electrode (7) or a combination of an immobilized enzyme column (17) and an electrode (16) sensitive to an enzyme reaction product, and

   - determination means (14) for determining the average molecular weight (Ma), the average chain length (Dp) and/or the DE value from the aldose and particularly the glucose amount (Gf; Gt; Gr) or respective data measured by the measuring means (7; 16, 17) in accordance with predetermined relations (Figs. 1, 3).

10. The method according to claim 9, characterized in that the determination means (14) of the apparatus used are designed such as to calculate the average molecular weight (Ma), the average chain length (Dp) and/or the dextrose equivalent (DE) value according to the following equations [2], [3] or [4]:

$$Dp = 2 \cdot Gt/(Gt - Gr) \qquad [2]$$

$$Ma = 162 \cdot Dp \qquad [3]$$

$$DE = 100(Gt - Gr)180/2 \cdot 162 \cdot Gt \qquad [4].$$

11. The method according to claim 9, characterized in that the determination means (14) of the apparatus used are designed such as to calculate the average molecular weight (Ma), the average chain length (Dp) and/or the dextrose equivalent (DE) value according to the following equations [6], [7], [9] or [10] :

$$Dp = 2 \cdot Gt/(Gt - Gr + Gf) \qquad [6]$$

$$Ma = 18(19 \cdot Gt + Gf - Gr)/(Gt - Gr + Gf) \qquad [7]$$

$$DE = 1000(Gt - Gr + Gf)/(19 \cdot Gt - Gr + Gf) \qquad [9]$$

$$DE = 1000/(9 \cdot Dp + 1) \qquad [10].$$

**12.** The method according to claims 9 to 11, characterized in that the determination means (14) of the apparatus used comprise a computer, particularly a personal computer, or a microprocessor system.

**13.** The method according to claims 9 to 12, characterized in that the apparatus used has at least one of the following features:

- The apparatus is of batch type, flow type or stopped-flow type;

- the measuring means (7; 16, 17) are provided in a thermostat (10);

- the measuring means (7; 8, 9, 10; 8, 9, 10, 16, 17) comprise an immobilized enzyme electrode (7), a reference electrode (8), a counter electrode (9) and a potentiostat (11), or comprise an immobilized enzyme column (17), a reference electrode (8), a counter electrode (9), a $H_2O_2$ electrode (16) or an $O_2$ electrode (16) and a potentiostat (11);

- the measuring means (7; 16, 17) comprise a flow pipe (4) provided upstream and comprising an injector (3) at its intake end;

- the immobilized enzyme electrode (7) or the immobilized enzyme column (17) comprise immobilized galactose oxidase, and glucose oxidase or pyranose oxidase;

- the electrode (16) is an $H_2O_2$ or an $O_2$ electrode;

- the electrodes (7; 16), the reference electrode (8) and the counter electrode (9) are provided in a measurement cell (5);

- the determination means (14) comprise an A/D converter for A/D converting the data from the potentiostat to be input into the computer or microprocessor system (14),

- the computer or microprocessor system (14) comprises means for storing, transferring output and/or displaying data and/or results, particularly Ma, Dp and/or DE values;

- the determination means (14) comprise a printer (15) for printing data and/or results, particularly Ma, Dp and/or DE values;

- the computer (14) comprises a keyboard for data input and operation;

- the apparatus further comprises means for hydrolyzing the samples before the aldose and particularly glucose measurement;

- the apparatus further comprises means for the reduction of reducing terminal groups of the samples;

- the apparatus is automatized in such a manner that the values of Gt and Gr, and eventually also of Gf, are automatically determined from one or more samples, and the results (Ma, Dp, DE value) are automatically calculated and output therefor.

**Patentansprüche**

**1.** Verfahren zur Bestimmung des mittleren Molekulargewichts (Ma), der mittleren Kettenlänge (Dp) und/oder des Wertes des Dextroseäquivalents (DE) von Oligosacchariden oder Polysacchariden, die Aldose-Zuckermonomereinheiten enthalten, insbesondere Glucose- oder Galactoseeinheiten, und/oder von verwandten Kohlenhydraten, das folgende Schritte umfaßt:

(A) quantitative Bestimmung der Aldose-Gesamtmenge (Gt) einer hydrolysierten Probe mit einer Enzymelektrode mit immobilisiertem Enzym oder einer Kombination einer Säule mit immobilisiertem Enzym und einer Elektrode, die auf ein Enzym-Reaktionsprodukt anspricht,

(B) Reduktion der reduzierenden Endgruppen einer Probe und quantitative Bestimmung der Aldosemenge (Gr) der hydrolysierten Probe mit einer Enzymelektrode mit immobilisiertem Enzym oder einer Kombination einer Säule mit immobilisiertem Enzym und einer Elektrode, die auf ein Enzym-Reaktionsprodukt anspricht, und

(C) Berechnung des mittleren Molekulargewichts (Ma), der mittleren Kettenlänge (Dp) und/oder des Wertes des Dextroseäquivalents (DE) aus der Aldose-Gesamtmenge (Gt) und der Aldosemenge (Gr) aufgrund vorgegebener Beziehungen.

2. Verfahren nach Anspruch 1 zur Analyse von Oligosacchariden oder Polysacchariden, die Glucoseeinheiten enthalten oder aus ihnen bestehen, insbesondere von Stärke und/oder verwandten Kohlenhydraten, das folgende Schritte umfaßt:

(A) quantitative Bestimmung der Glucose-Gesamtmenge (Gt) einer hydrolysierten Probe mit einer Enzymelektrode mit immobilisiertem Enzym oder einer Kombination einer Säule mit immobilisiertem Enzym und einer Elektrode, die auf ein Enzym-Reaktionsprodukt anspricht,

(B) Reduktion der reduzierenden Endgruppen einer Probe und quantitative Bestimmung der Glucosemenge (Gr) der hydrolysierten Probe mit einer Enzymelektrode mit immobilisiertem Enzym oder einer Kombination einer Säule mit immobilisiertem Enzym und einer Elektrode, die auf ein Enzym-Reaktionsprodukt anspricht, und

(C) Berechnung des mittleren Molekulargewichts (Ma), der mittleren Kettenlänge (Dp) und/oder des Wertes des Dextroseäquivalents (DE) aus der Glucose-Gesamtmenge (Gt) und der Glucosemenge (Gr) aufgrund vorgegebener Beziehungen.

3. Verfahren nach den Ansprüchen 1 und 2, wobei in Schritt B zur Reduktion der reduzierenden Endgruppen der Probe ein Borhydrid, vorzugsweise Natriumborhydrid und/oder Kaliumborhydrid, verwendet wird.

4. Verfahren nach den Ansprüchen 1 bis 3, wobei zur Hydrolyse der Probe vor oder nach der Reduktion der reduzierenden Endgruppen eine Amylase, vorzugsweise eine $\alpha$-Amylase, und/oder eine Glucoamylase und/oder eine Glucosidase, vorzugsweise eine $\alpha$-Glucosidase, verwendet werden.

5. Verfahren nach den Ansprüchen 2 bis 4, dadurch gekennzeichnet, daß für Proben, in denen keine freie Glucose enthalten ist, und im Fall der Verwendung einer Glucoamylase zur Hydrolyse die mittlere Kettenlänge (Dp), das mittlere Molekulargewicht (Ma) bzw. der Wert des Dextroseäquivalents (DE) nach den nachstehenden Gleichungen [2], [3] bzw. [4] berechnet werden:

$$Dp = 2 \cdot Gt/(Gt - Gr) \qquad [2]$$

$$Ma = 162 \cdot Dp \qquad [3]$$

$$DE = 100(Gt - Gr)180/2 \cdot 162 \cdot Gt \qquad [4].$$

6. Verfahren nach den Ansprüchen 1 bis 4, gekennzeichnet durch quantitative Bestimmung der in der Probe enthaltenen Menge an freier Aldose bzw. freier Glucose (Gf), die vor Schritt A oder unabhängig davon und Berechnung des mittleren Molekulargewichts (Ma), der mittleren Kettenlänge (Dp) und/oder des Wertes des Dextroseäquivalents (DE) aus der Menge an freier Aldose bzw. freier Glucose (Gf), der Aldose- oder Glucose-Gesamtmenge (Gt) und der Aldose- oder Glucosemenge (Gr).

7. Verfahren nach den Ansprüchen 1 bis 4 und 6, dadurch gekennzeichnet, daß im Fall von Oligo- und Polysacchariden auf der Basis von Glucose in Schritt C das mittlere Molekulargewicht (Ma), die mittlere Kettenlänge (Dp) bzw. der Wert des Dextroseäquivalents (DE) nach den nachstehenden Gleichungen [6], [7], [9] bzw. [10] berechnet werden:

$$Dp = 2 \cdot Gt/(Gt - Gr + Gf) \tag{6}$$

$$Ma = 18(19 \cdot Gt + Gf - Gr)/(Gt - Gr + Gf) \tag{7}$$

$$DE = 1000(Gt - Gr + Gf)/(19 \cdot Gt - Gr + Gf) \tag{9}$$

$$DE = 1000/(9 \cdot Dp + 1) \tag{10}.$$

8. Verfahren nach den Ansprüchen 1 bis 7, dadurch gekennzeichnet, daß die Aldose- bzw. Glucose-Gesamtmenge (Gt) und/oder die Aldose- bzw. Glucosemenge (Gr) in den Schritten A und/oder B nach der Hydrolyse der entsprechenden Proben mit einer Enzymelektrode mit immobilisiertem Enzym zur Erfassung der Aldose, insbesondere der Glucose, die eine immobilisierte Aldoseoxidase und insbesondere eine immobilisierte Glucoseoxidase oder Pyranoseoxidase enthält, und/oder unter Verwendung einer Säule mit einem immbolisierten Enzym, die eine immobilisierte Aldoseoxidase und insbesondere eine immobilisierte Glucoseoxidase oder Pyranoseoxidase enthält, und einer Wasserstoffperoxidelektrode oder einer Sauerstoffelektrode bestimmt werden.

9. Verfahren nach den Ansprüchen 1 bis 8, das durchgeführt wird mit einer Vorrichtung, die aufweist:

-   Eine Meßeinrichtung (7; 16, 17) zur quantitativen elektrochemischen Bestimmung der Menge an Aldose und insbesondere an Glucose (Gf; Gt; Gr) einer Probe, die eine Enzymelektrode mit immobilisiertem Enzym (7) oder eine Kombination einer Säule mit immobilisiertem Enzym (17) und einer Elektrode (16), die auf ein Enzymreaktionsprodukt anspricht, aufweist, und

-   eine Bestimmungseinrichtung (14) zur Bestimmung des mittleren Molekulargewichts (Ma), der mittleren Kettenlänge (Dp) und/oder des Wertes (DE) aus der Aldosemenge und insbesondere der Glucosemenge (Gf; Gt; Gr) oder entsprechender Daten, die mit der Meßeinrichtung (7; 16, 17) gemessen wurden, aufgrund vorgegebener Relationen (Fig. 1, 3).

10. Verfahren nach Anspruch 9, dadurch gekennzeichnet, daß die Bestimmungseinrichtung (14) der verwendeten Vorrichtung so ausgelegt ist, daß sie das mittlere Molekulargewicht (Ma), die mittlere Kettenlänge (Dp) und/oder den Wert des Dextroseäquivalents (DE) nach den nachstehenden Gleichungen [2], [3] bzw. [4] berechnet:

$$Dp = 2 \cdot Gt/(Gt - Gr) \tag{2}$$

$$Ma = 162 \cdot Dp \tag{3}$$

$$DE = 100(Gt - Gr)180/2 \cdot 162 \cdot Gt \tag{4}.$$

11. Verfahren nach Anspruch 9, dadurch gekennzeichnet, daß die Bestimmungseinrichtung (14) der verwendeten Vorrichtung so ausgelegt ist, daß sie das mittlere Molekulargewicht (Ma), die mittlere Kettenlänge (Dp) und/oder den Wert des Dextroseäquivalents (DE) nach den nachstehenden Gleichungen [6], [7], [9] bzw. [10] berechnet:

$$Dp = 2 \cdot Gt/(Gt - Gr + Gf) \tag{6}$$

$$Ma = 18(19 \cdot Gt + Gf - Gr)/(Gt - Gr + Gf) \tag{7}$$

$$DE = 1000(Gt - Gr + Gf)/(19 \cdot Gt - Gr + Gf) \tag{9}$$

$$DE = 1000/(9 \cdot Dp + 1) \tag{10}.$$

12. Verfahren nach den Ansprüchen 9 bis 11, dadurch gekennzeichnet, daß die Bestimmungseinrichtung (14) der verwendeten Vorrichtung einen Computer, insbesondere einen Personal Computer, oder ein Mikroprozessorsystem aufweist.

**13.** Verfahren nach den Ansprüchen 9 bis 12, dadurch gekennzeichnet, daß die verwendete Vorrichtung mindestens eines der nachstehenden Merkmale aufweist:

- Die Vorrichtung ist eine absatzweise arbeitende Vorrichtung, eine Vorrichtung vom Strömungstyp oder eine Vorrichtung vom Stopped-flow-Typ;

- die Meßeinrichtung (7; 16, 17) ist in einem Thermostaten (10) vorgesehen);

- die Meßeinrichtung (7; 8, 9, 10; 8, 9, 10, 16, 17) weist eine Enzymelektrode mit immobilisiertem Enzym (7), eine Bezugselektrode (8), eine Gegenelektrode (9) und einen Potentiostaten (11) oder eine Säule mit immobilisiertem Enzym (17), eine Bezugselektrode (8), eine Gegenelektrode (9), eine $H_2O_2$-Elektrode (16) oder eine 02-Elektrode (16) und einen Potentiostaten (11) auf;

- die Meßeinrichtung (7; 16, 17) umfaßt ein Strömungsrohr (4), das stromauf von ihr vorgesehen ist und eine Einspritzvorrichtung (3) an seinem eingangsseitigen Ende aufweist;

- die Enzymelektrode mit immobilisiertem Enzym (7) oder die Säule mit immobilisiertem Enzym (17) enthält immobilisierte Galactoseoxidase und Glucoseoxidase oder Pyranoseoxidase;

- die Elektrode (16) ist eine $H_2O_2$-Elektrode oder eine $O_2$-Elektrode;

- die Elektroden (7; 16), die Bezugselektrode (8) und die Gegenelektrode (9) sind in einer Meßzelle (5) vorgesehen;

- die Bestimmungseinrichtung (14) umfaßt einen A/D-Wandler zur A/D-Wandlung der Daten vom Potentiostaten zur Eingabe in den Computer oder das Mikroprozessorsystem (14);

- der Computer oder das Mikroprozessorsystem (14) umfassen eine Einrichtung zur Speicherung, Übertragung, Ausgabe und/oder Anzeige von Daten und/oder Ergebnissen, insbesondere der Werte für Ma, Dp und/oder DE;

- die Bestimmungseinrichtung (14) umfaßt einen Drucker (15) zum Ausdruck von Daten und/oder Ergebnissen, insbesondere der Werte für (Ma), (Dp) und/oder (DE);

- der Computer (14) weist ein Keyboard zur Dateneingabe und zur Betätigung auf;

- die Vorrichtung umfaßt ferner eine Einrichtung zur Hydrolyse der Proben vor der Messung der Aldose und insbesondere der Glucose;

- die Vorrichtung umfaßt ferner eine Einrichtung zur Reduktion reduzierender Endgruppen der Proben;

- die Vorrichtung ist in der Weise automatisiert, daß die Werte von (Gt) und (Gr) und gegebenenfalls auch von (Gf) von einer oder mehreren Proben automatisch bestimmt und die Ergebnisse (Werte von Ma, Dp, DE) automatisch berechnet und entsprechend ausgegeben werden.

**Revendications**

**1.** Méthode pour la détermination de la masse moléculaire moyenne (Ma), de la longueur moyenne des chaînes (Dp) et/ou de la valeur de l'équivalent en dextrose (DE) d'oligosaccharides ou de polysaccharides contenant, ou étant constitués par, des unités monomères aldose, en particulier des unités glucose ou galactose, et/ou des hydrates de carbone analogues,

comprenant les étapes suivantes :

(A) la détermination quantitative de la quantité totale d'aldose (Gt) d'un échantillon hydrolysé au moyen d'une électrode à enzyme immobilisé ou d'une combinaison d'une colonne à enzyme immobilisé et d'une électrode sensible à un produit de la réaction enzymatique,

(B) la réduction des groupes terminaux réducteurs d'un échantillon et la détermination quantitative de la quantité d'aldose (Gr) de l'échantillon hydrolysé par une électrode à enzyme immobilisé ou une combinaison d'une colonne à enzyme immobilisé et d'une électrode sensible à un produit de la réaction enzymatique, et

(C) le calcul de la masse moléculaire moyenne (Ma), de la longueur moyenne des chaînes (Dp) et/ou de la valeur de l'équivalent en dextrose (DE) à partir de la quantité totale d'aldose (Gt) et de la quantité d'aldose (Gr) en utilisant des relations prédéterminées.

2.  Méthode selon la revendication 1, pour l'analyse d'oligosaccharides ou de polysaccharides contenant, ou étant constitués par, des unités glucose, et en particulier d'amidon et/ou d'hydrates de carbone analogues,
    comprenant les étapes suivantes :

    (A) la détermination quantitative de la quantité totale de glucose (Gt) d'un échantillon hydrolysé au moyen d'une électrode à enzyme immobilisé ou d'une combinaison d'une colonne à enzyme immobilisé et d'une électrode sensible à un produit de la réaction enzymatique,
    (B) la réduction des groupes terminaux réducteurs d'un échantillon et la détermination quantitative de la quantité de glucose (Gr) de l'échantillon hydrolysé par une électrode à enzyme immobilisé ou une combinaison d'une colonne à enzyme immobilisé et d'une électrode sensible à un produit de la réaction enzymatique, et

    (C) le calcul de la masse moléculaire moyenne (Ma), de la longueur moyenne des chaînes (Dp) et/ou de la valeur de l'équivalent en dextrose (DE) à partir de la quantité totale de glucose (Gt) et de la quantité de glucose (Gr) à partir de relations prédéterminées.

3.  Méthode selon les revendications 1 et 2, dans laquelle un borohydrure, de préférence un borohydrure de sodium et/ou de potassium, est utilisé dans l'étape B pour la réduction des groupes réducteurs terminaux de l'échantillon.

4.  Méthode selon les revendications 1 à 3, dans laquelle une amylase, de préférence une $\alpha$-amylase, et/ou une glucosamylase et/ou une glucosidase, de préférence une $\alpha$-glucosidase, sont utilisées pour l'hydrolyse de l'échantillon avant ou après la réduction des groupes réducteurs terminaux.

5.  Méthode selon les revendications 2 à 4, caractérisée en ce que, pour des échantillons ne contenant pas de glucose libre, et dans le cas où l'on utilise la glucosamylase pour l'hydrolyse, la longueur moyenne des chaînes (Dp), la masse moléculaire moyenne (Ma) ou la valeur de l'équivalent en dextrose (DE) sont calculées en utilisant les équations [2], [3] ou [4] suivantes :

$$Dp = 2 \cdot Gt/(Gt\text{-}Gr) \qquad\qquad [2]$$

$$Ma = 162 \cdot Dp \qquad\qquad [3]$$

$$DE = 100 \, (Gt\text{-}Gr) \, 180/2 \cdot 162 \cdot Gt \qquad\qquad [4]$$

6.  Méthode selon les revendications 1 à 4, caractérisée par la détermination quantitative de la quantité d'aldose ou de glucose libre (Gf), respectivement, contenue dans l'échantillon avant l'étape A ou indépendamment et le calcul de la masse moléculaire moyenne (Ma), de la longueur moyenne de la chaîne (Dp) et/ou de la valeur de l'équivalent en dextrose (DE) à partir de la quantité d'aldose ou de glucose libre (Gf), de la quantité totale d'aldose ou de glucose (Gt) et de la quantité d'aldose ou de glucose (Gr).

7.  Méthode selon les revendications 1 à 4 et 6, caractérisée en ce que, dans le cas d'oligo- et polysaccharides à base de glucose, dans l'étape C, la masse moléculaire moyenne (Ma), la longueur moyenne des chaînes (Dp) ou la valeur de l'équivalent en dextrose (DE) sont calculées en utilisant les équations [6], [7], [9] ou [10] suivantes :

$$Dp = 2 \cdot Gt/(Gt\text{-}Gr\text{+}Gf) \qquad\qquad [6]$$

$$Ma = 18(19 \cdot Gt + Gf \text{ - } Gr)/(Gt \text{ -}Gr\text{+}Gf) \qquad\qquad [7]$$

$$DE = 1000 \, (Gt \text{ - } Gr + Gf/(19 \cdot Gt \text{ -}Gr\text{+}Gf) \qquad\qquad [9]$$

$$DE = 1000/(9 \cdot Dp + 1) \qquad\qquad [10].$$

**8.** Méthode selon les revendications 1 à 7, caractérisée en ce que la quantité totale d'aldose ou de glucose (Gt), respectivement, et/ou la quantité d'aldose ou de glucose (Gr) sont déterminées dans les étapes A et/ou B après hydrolyse des échantillons respectifs, en utilisant une électrode à enzyme immobilisé pour la détection des aldoses, en particulier du glucose, contenant une aldose oxydase immobilisée, et en particulier une glucose oxydase ou une pyrannose oxydase immobilisée, et/ou en utilisant une colonne à enzyme immobilisé contenant une aldose oxydase immobilisée, et en particulier une glucose oxydase ou une pyrannose oxydase immobilisée, et une électrode à peroxyde d'hydrogène ou une électrode à oxygène.

**9.** Méthode selon les revendications 1 à 8 mise en oeuvre avec un appareil comprenant

- un moyen de mesure (7 ; 16, 17) pour la détermination quantitative électrochimique de la quantité d'aldose, et en particulier de la quantité de glucose, (Gf ; Gt ; Gr) d'un échantillon, constitué par une électrode à enzyme immobilisé (7) ou une combinaison d'une colonne à enzyme immobilisé (17) et d'une électrode (16) sensible à un produit de la réaction enzymatique,
et
- un moyen de détermination (14) pour déterminer la masse moléculaire moyenne (Ma), la longueur moyenne des chaînes (Dp) et/ou la valeur DE à partir de la quantité d'aldose, et en particulier de glucose, (Gf ; Gt ; Gr) ou des données respectives mesurées par le moyen de mesure (7 ; 16, 17) en utilisant des relations prédéterminées (figures 1, 3).

**10.** Méthode selon la revendication 9, caractérisée en ce que le moyen de détermination (14) de l'appareil utilisé est conçu de telle façon qu'il permet de calculer la masse moléculaire moyenne (Ma), la longueur moyenne des chaînes (Dp), et/ou la valeur de l'équivalent en dextrose (DE) en utilisant les équations [2], [3] ou [4] suivantes :

$$Dp = 2 \cdot Gt/(Gt\text{-}Gr) \qquad [2]$$

$$Ma = 162 \cdot Dp \qquad [3]$$

$$DE = 100 \, (Gt\text{-}Gr) \, 180/2 \cdot 162 \cdot Gt \qquad [4]$$

**11.** Méthode selon la revendication 9, caractérisée en ce que le moyen de détermination (14) de l'appareil utilisé est conçu pour calculer la masse moléculaire moyenne (Ma), la longueur moyenne des chaînes (Dp), et/ou la valeur de l'équivalent en dextrose (DE) à partir des équations [6], [7], [9] ou [10] suivantes :

$$Dp = 2 \cdot Gt/(Gt\text{-}Gr\text{+}Gf) \qquad [6]$$

$$Ma = 18(19 \cdot Gt + Gf \text{ - } Gr)/(Gt\text{-}Gr\text{+}Gf) \qquad [7]$$

$$DE = 1000 \, (Gt\text{-}Gr\text{+}Gf)/(19 \cdot Gt\text{-}Gr\text{+}Gf) \qquad [9]$$

$$DE = 1000/(9 \cdot Dp + 1) \qquad [10].$$

**12.** Méthode selon les revendications 9 à 11, caractérisée en ce que le moyen de détermination (14) de l'appareil utilisé est constitué par un ordinateur, en particulier un micro-ordinateur, ou un système à microprocesseur.

**13.** Méthode selon les revendications 9 à 12, caractérisée en ce que l'appareil utilisé a au moins une des caractéristiques suivantes :

- l'appareil est du type discontinu, continu ou à circulation étagée ;
- le moyen de mesure (7 ; 16, 17) est contenu dans une enceinte thermostatée (10);
- le moyen de mesure (7 ; 8, 9, 10 ; 8, 9, 10, 16, 17) comprend une électrode à enzyme immobilisé (7), une électrode de référence (8), une électrode antagoniste (9) et un potentiostat (11),
ou comprennent une colonne à enzyme immobilisé (17), une électrode de référence (8), une électrode antagoniste (9), une électrode à $H_2O_2$ (16) ou une électrode à $O_2$ (16) et un potentiostat (11)
- le moyen de mesure (7 ; 16, 17) comprend un tube d'écoulement (4) situé en amont et comprenant un injecteur (3) à son extrémité d'admission ;
- l'électrode à enzyme immobilisé (7) ou la colonne à enzyme immobilisé (17) contiennent une galactose oxydase et une glucose oxydase ou une pyrannose oxydase immobilisées ;

- l'électrode (16) est une électrode à $H_2O_2$ ou une électrode à $O_2$ ;
- les électrodes (7 ; 16), l'électrode de référence (8) et l'électrode antagoniste (9) sont contenues dans une cellule de mesure (5);
- le moyen de détermination (14) contient un convertisseur analogique/numérique pour la conversion analogique/numérique des données du potentiostat avant leur introduction dans l'ordinateur ou le système à microprocesseur (14),
- l'ordinateur ou le système à microprocesseur (14) comprend des moyens de stockage, de transfert des sorties et/ou d'affichage des données et/ou des résultats, en particulier des valeurs de Ma, Dp et/ou DE.
- le moyen de détermination (14) contient une imprimante (15) pour imprimer les données et/ou les résultats, en particulier les valeurs de Ma, Dp et/ou DE ;
- l'ordinateur (14) comprend un clavier pour la saisie des données et pour le fonctionnement ;
- l'appareil comprend en outre des moyens d'hydrolyse des échantillons avant la mesure des aldoses et en particulier du glucose ;
- l'appareil comprend en outre des moyens pour la réduction des groupes terminaux réducteurs des échantillons ;
- l'appareil est automatisé de telle façon que les valeurs de Gt et Gr, et éventuellement aussi de Gf, soient automatiquement déterminées sur un ou plusieurs échantillons et que les résultats (Ma, Dp, valeur de DE) soient automatiquement calculés et sortis.

Fig. 1

EP 0 451 811 B1

# Fig.2

START

INPUT SEQUENCE OF MEASUREMENT

MEASUREMENT OF GLUCOSE AMOUNT

TEMPORARY STORAGE OF DATA?

MEASUREMENT FINISHED?

NO

YES

CALCULATION

PRINTING OF RESULT

END?

NO

YES

END

Fig. 3